# EUROPEAN PATENT APPLICATION

(11) **EP 4 578 575 A1**
(43) Date of publication of application: **02.07.2025**
(21) Application number: 22956438.0
(22) Date of filing: 23.08.2022
(51) Int. Cl.: B22F 9/24

(54) **METHOD FOR PRODUCING PLATINUM NANO-PARTICLE SOLUTION, ANTIBACTERIAL AGENT, AND ANTIBACTERIAL PRODUCT**

(71) Applicant: CEP Project Co., Ltd., Tokyo 132-0035 (JP)
(72) Inventor: HONTANI, Kenro, Tokyo 132-0035 (JP); KAMEI, Takeshi, Tokyo 132-0035 (JP)
(74) Representative: Schön, Christoph
(86) International application number: PCT/JP2022/031720
(87) International publication number: WO 2024/042610

(57) **Abstract**

A method for producing a platinum nanoparticle solution, including preparing platinum nanoparticles by adding a reducing agent to a solution containing platinum ions derived from chloroplatinic acid or a chloroplatinic acid salt, to subject the platinum ions to reduction treatment; and adding an inorganic acid. An antibacterial product agent in which a platinum nanoparticle solution containing platinum nanoparticles, an inorganic acid and water is used, and an antibacterial product in which the antibacterial agent is applied.

## Description

### Technical Field

One of embodiments of the present invention relates to a method for producing a platinum nanoparticle solution. Other embodiments of the present invention relate to an antibacterial agent and an antibacterial product in which platinum nanoparticles are used.

### Background Art

Metal nanoparticles are metal particles having an average particle diameter typically of 1 to 100 nm, and have properties different from those of bulk metals, and thus recently have attracted attention in various applications including electronic materials, electrode materials, and catalyst materials. Among these, noble metal nanoparticles are excellent in ability to adsorb a finely divided material, and are known to have functional properties such as an antibacterial property, a deodorant property and an antiviral property. Therefore, in recent years, various studies using noble metal nanoparticles have been conducted for a wide range of applications related to food, clothing and housing.

For example, liquid preparations containing nanoparticles of a noble metal such as gold, silver or copper, or antibacterial products to which noble metal nanoparticles are attached have been heretofore known. On the other hand, in recent years, platinum nanoparticles have attracted attention because they can maintain functional properties such as an antibacterial property for a long period, and have high safety for human bodies. Development of liquid preparations such as antibacterial agents, humidifiers, air cleaners, antibacterial products and the like, in which platinum nanoparticles are used, is proceeding.

A liquid preparation containing platinum nanoparticles is prepared as a colloidal solution in which platinum nanoparticles are uniformly dispersed in a solvent such as water (hereinafter, referred to as a "platinum nanoparticle solution"). The method for producing platinum nanoparticles is not limited. A method known as a method for producing metal nanoparticles can be applied. Specifically, the platinum nanoparticles may be produced by either a breakdown method such as dry grinding or wet grinding, or a buildup method such as a liquid phase method or a gas phase method. Among these, a liquid phase method is preferably applied from the viewpoint of easily obtaining a platinum nanoparticle solution.

In the production of platinum nanoparticles by a liquid phase method, a reducing agent is added to a solution containing platinum ions derived from a platinic acid salt or the like, so that the platinum ions are reduced to form platinum nanoparticles. Therefore, the reaction solution in the production of platinum nanoparticles can be directly used as a platinum nanoparticle solution. On the other hand, the platinum nanoparticle solution often contains a protective agent such as a surfactant or a polymer compound, which attaches to the surface of the platinum nanoparticles, and acts to suppress aggregation of the platinum nanoparticles and maintain dispersibility (e.g., Patent Literature 1).

### Citation List

### Patent Literature

Patent Literature 1: JP 2002-001095 A

### Summary of Invention

### Technical Problem

However, for example, if the platinum nanoparticle solution is used for antibacterial agents and the like, the adsorbing ability of platinum nanoparticles may be reduced by a surfactant or a protective agent, resulting in deterioration of functional properties such as an antibacterial property. In addition, the human body may be affected depending on the type of surfactant or protective agent. For these reasons, a platinum nanoparticle solution having further excellent functional properties and safety for human bodies is desired for a wide range of applications related to food, clothing and housing.

On the other hand, it is preferable to increase the content of the platinum nanoparticles in the platinum nanoparticle solution from the viewpoint of easily obtaining functions and effects of platinum nanoparticles such as an antibacterial property. However, if a surfactant or a protective agent is not used during preparation of the platinum nanoparticle solution, aggregation and precipitation are likely to occur as a time-dependent change with an increase in content of platinum nanoparticles. As a result, the platinum nanoparticle solution is likely to undergo a change in color from a substantially transparent state to light gray and to dark black (time-dependent discoloration).

When an antibacterial agent is prepared using a platinum nanoparticle solution, the platinum nanoparticle solution is typically used in an undiluted form, or used after being diluted with a solvent such as water if necessary. However, as described above, if time-dependent discoloration of the platinum nanoparticle solution proceeds, defects are likely to occur in application to a white material or the like. For example, if a discolored platinum nanoparticle solution is applied to a white material such as paper, cloth or fiber, defects such as staining (blackening) of the solution are likely to occur. Therefore, for extending the range of application of antibacterial agents, it is necessary to improve the time-dependent discoloration of the platinum nanoparticle solution.

Accordingly, in view of the above-described circumstances, one of embodiments of the present invention provides a method for producing a platinum nanoparticle solution capable of resisting time-dependent discoloration. Other embodiments of the present invention provide an antibacterial agent and an antibacterial product which have excellent functions and effects and are capable of resisting time-dependent discoloration, with the use of the platinum nanoparticle solution.

### Solution to Problem

The present inventors have intensively conducted studies to solve the above-described problems, and completed the present invention. That is, some embodiments of the present invention relate to the following. However, the present invention is not limited to the embodiments described below, and includes various embodiments.

One of embodiments of the present invention relates to a method for producing a platinum nanoparticle solution, including adding a reducing agent to a solution containing platinum ions derived from chloroplatinic acid or a chloroplatinic acid salt, to subject the platinum ions to reduction treatment, and adding an inorganic acid.

[1] A method for producing a platinum nanoparticle solution, including: adding a reducing agent to a solution containing platinum ions derived from chloroplatinic acid or a chloroplatinic acid salt, to subject the platinum ions to reduction treatment, and adding an inorganic acid.
[2] The method for producing a platinum nanoparticle solution according to [1], in which the chloroplatinic acid salt contains at least one selected from the group consisting of sodium chloroplatinate, potassium chloroplatinate, platinum (II) sodium chloride, platinum (II) potassium chloride, and ammonium chloroplatinate.
[3] The method for producing a platinum nanoparticle solution according to [1], in which a pH of the platinum nanoparticle solution is adjusted within a range of 4 to 6 by the addition of the inorganic acid.
[4] The method for producing a platinum nanoparticle solution according to any one of [1] to [3], in which the inorganic acid is dilute hydrochloric acid.
[5] The method for producing a platinum nanoparticle solution according to any one of [1] to [4], in which the reducing agent contains at least one selected from the group consisting of ascorbic acid or an ascorbic acid salt, arginine acid or an arginine acid salt, a dicarboxylic acid, an alcohol, and a polysaccharide.
[6] The method for producing a platinum nanoparticle solution according to any one of [1] to [5], in which in the reduction treatment, citric acid or a citric acid salt is further added.
[7] An antibacterial agent including: platinum nanoparticles, an inorganic acid, and water.
[8] The antibacterial agent according to [7], in which the inorganic acid is dilute hydrochloric acid.
[9] The antibacterial agent according to [7] or [8], further including chloroplatinic acid or a chloroplatinic acid salt.
[10] The antibacterial agent according to any one of [7] to [9], further including potassium iodide.
[11] A coating material including: the antibacterial agent according to any one of [7] to [10].
[12] An antibacterial product in which the antibacterial agent according to any one of [7] to [10] or the coating material according to [11] is applied to a daily commodity made from a material selected from the group consisting of synthetic or natural fiber, plastic, ceramic, and wood.
[13] The antibacterial product according to [12], in which the antibacterial product is a mask, gauze, a toothbrush, a cup, or an antiseptic wipe.

### Advantageous Effects of Invention

According to one of embodiments of the present invention, a method for producing a platinum nanoparticle solution capable of resisting time-dependent discoloration can be provided. In addition, an antibacterial agent and an antibacterial product capable of resisting time-dependent discoloration can be provided with the use of the platinum nanoparticle solution.

### Brief Description of Drawings

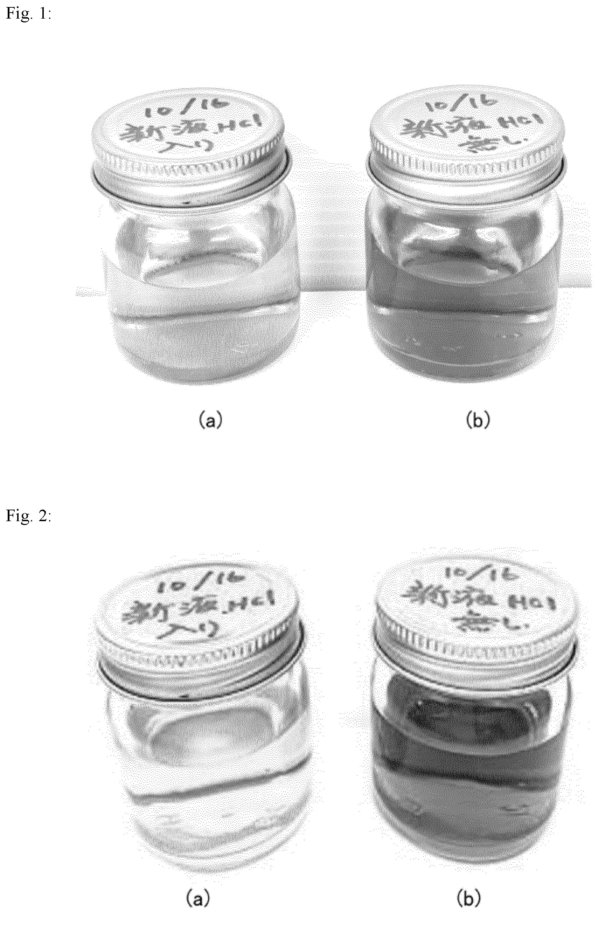

[Fig. 1] Fig. 1 illustrates pictures illustrating states of time-dependent discoloration of antibacterial agents containing platinum nanoparticles (after storage for 4 months), where picture (a) corresponds to an antibacterial agent of Example 1 (with addition of dilute hydrochloric acid), and picture (b) corresponds to an antibacterial agent of Comparative Example 1 (without addition of dilute hydrochloric acid).
[Fig. 2] Fig. 2 illustrates pictures illustrating states of time-dependent discoloration of antibacterial agents containing platinum nanoparticles (after storage for 6 months), where picture (a) corresponds to an antibacterial agent of Example 1 (with addition of dilute hydrochloric acid), and picture (b) corresponds to an antibacterial agent of Comparative Example 1 (without addition of dilute hydrochloric acid).

### Description of Embodiments

Hereinafter, some embodiments of the present invention will be described. However, the present invention is not limited to the following embodiments.

### (Method for producing platinum nanoparticle solution)

One of embodiments relates to a method for producing a platinum nanoparticle solution. The production method includes adding a reducing agent to a solution containing platinum ions derived from chloroplatinic acid or a chloroplatinic acid salt, to subject the platinum ions to reduction treatment, and adding an inorganic acid.

According to some of the above embodiments, by adding an inorganic acid to the solution containing platinum nanoparticles obtained by the reduction treatment, an undesirable time-dependent change of the platinum nanoparticle solution such as discoloration associated with storage can be improved. Thus, the platinum nanoparticle solution after storage can be preferably used as a liquid preparation such as an antibacterial agent in an undiluted form or after being diluted with a solvent such as water if necessary. The water used for dilution is preferably deionized water.

The amount of the inorganic acid added to the solution containing platinum nanoparticles is not limited, but is preferably adjusted so that the pH of the solution after the reduction treatment is in a range where the solution is weekly acidic. In one of embodiments, the pH of the solution after the reduction treatment may be 4 or more, and may be 6 or less. When the pH of the solution containing platinum nanoparticles after the reduction treatment is adjusted to fall within the range of 4 to 6, aggregation of the platinum nanoparticles (enlargement of particles) is easily suppressed. In addition, an excellent antiseptic effect can be easily obtained when the antibacterial agent is prepared using the above solution. Further, when the solution is sprayed or applied, an undesirable impact on the object can be easily suppressed.

In the production method of some of the above embodiments, the pH of the solution after the reduction treatment may be preferably in the range of 4.2 to 5.8, more preferably in the range of 4.3 to 5.6, still more preferably in the range of 4.4 to 5.4. In one of embodiments, the pH of the solution may be still more preferably in the range of 4.4 to 5.0.

The inorganic acid is not limited as long as the pH of the solution can be adjusted. For example, hydrochloric acid can be used as the inorganic acid, and dilute hydrochloric acid can be preferably used. The dilute hydrochloric acid may be dilute hydrochloric acid available as a commercial product. The commercially available dilute hydrochloric acid has a hydrogen chloride concentration of about 10 mass%. Thus, an aqueous solution in which the concentration of hydrogen chloride is adjusted to 9.5 to 10.5 mass% may be used.

As described later, the source of platinum ions may be preferably chloroplatinic acid or a chloroplatinic acid salt. For example, a solution of platinum (II) monopotassium chloride can be preferably used. In the solution of solution of platinum (II) monopotassium chloride, ionization of nanoplatinum reduces nano-Pt(II) to nano-Pt(0) as a time-dependent change, so that aggregation easily occurs. In the production method of some embodiments, to a solution in such a state is added an inorganic acid such as dilute hydrochloric acid, whereby time-dependent discoloration of the solution can be suppressed. Without being bound by theory, it is presumed to be because a disproportionation reaction represented by the following formula (1) is suppressed by addition of the inorganic acid, so that ionic bonding in the nano-Pt(II) state is maintained.

Formula (1) 2K₂[Pt(II)C1₄] → K₂[Pt(IV)C1₆] + Pt(0) + 2KCl

The average particle size of the platinum nanoparticles may be 100 nm or less. In one of embodiments, the average particle size of the platinum nanoparticles may be preferably 1 to 50 nm, more preferably 2 to 20 nm, still more preferably 5 to 10 nm. The average particle size of the platinum nanoparticles is preferably as small as possible from the viewpoint that the catalytic effect is improved as the contact area expands. When the average particle size of the platinum nanoparticles is adjusted within the above-described range, aggregation and precipitation of the platinum nanoparticles in water can be easily suppressed, and good dispersibility can be easily maintained. By adjusting conditions during production of platinum nanoparticles, platinum nanoparticles having a desired average particle size can be easily obtained.

From the viewpoint of the type of usage such as spraying and the raw material, the platinum nanoparticles are powder which is as small as possible as described above, and their particle sizes are preferably equalized. However, from the viewpoint of cost and availability, it is practically unnecessary to uniform the particle size, and the particle size may be un-uniform.

In some of the above embodiments, the solution containing platinum ions can be prepared by dissolving a water-soluble platinum complex in a solvent containing at least water. In one of embodiments, the water is preferably deionized water. The source of platinum ions may preferably be chloroplatinic acid or a chloroplatinic acid salt, which dissolves in water to form platinum ions. It is possible to use chloroplatinic acid or a chloroplatinic acid salt which is well known in the art. A chloroplatinic acid salt is more preferable.

Specific examples include chloroplatinic acid (H₂PtCl₆), chloroplatinic acid hexahydrate (H₂PtCl₆(H₂O)₆), sodium chloroplatinate (potassium hexachloroplatinate, Na₂PtCl₆), potassium chloroplatinate (potassium hexachloroplatinate, Na₂PtCl₆), platinum (II) sodium chloride (sodium tetrachloroplatinate, Na₂PtCl₄), platinum (II) potassium chloride (potassium tetrachloroplatinate, K₂PtCl₄), and ammonium chloroplatinate ((NH₄)₂PtCl₆).

In one of embodiments, at least one selected from the group consisting of sodium chloroplatinate, potassium chloroplatinate, platinum (II) sodium chloride, platinum (II) potassium chloride, and ammonium chloroplatinate can be preferably used, as chloroplatinic acid salt. The chloroplatinic acid or chloroplatinic acid salt may be obtained commercially, or prepared by a method well known in the art.

The reduction treatment for platinum ions can be performed by a method well known in the art. For example, first, a solution containing platinum ions is prepared by dissolving chloroplatinic acid or a chloroplatinic acid salt in a solvent containing at least water. In one of embodiments, the concentration of platinum ions in the solution may be, for example, 0.001 to 0.1 mol/L as calculated from the amount of the chloroplatinic acid or chloroplatinic acid salt used. The concentration of the platinum ions may be preferably 0.005 to 0.1 mol/L, more preferably 0.01 to 0.1 mol/L.

Next, a reducing agent or a solution containing a reducing agent is added to a solution containing platinum ions, and mixing is performed to subject platinum ions to reduction treatment, whereby a platinum nanoparticle solution can be obtained. The reduction treatment can be performed at room temperature or under heating.

The reducing agent that can be used for the reduction treatment in some of the above embodiments is not limited as long as it can reduce platinum ions. An organic compound having a reducing action can be preferably used. In one of embodiments, at least one selected from the group consisting of ascorbic acid or an ascorbic acid salt, arginine acid or an arginine acid salt, a dicarboxylic acid, an alcohol and a polysaccharide can be used as the reducing agent.

More specifically, the ascorbic acid may be L-ascorbic acid. The ascorbic acid salt may be, for example, sodium ascorbate, calcium ascorbate, potassium ascorbate, or the like. The arginine acid salt may be, for example, arginine acid sodium. The dicarboxylic acid may be, for example, formic acid, or oxalic acid. The alcohol may be glycose, tetraethylene glycol, ethanol, or the like. The polysaccharide may be, for example, xylitol, glycerol, or chitosan. Among these, ascorbic acid can be preferably used.

The amount of the reducing agent added may be preferably 2 to 10 times the concentration of platinum ions derived from chloroplatinic acid or chloroplatinic acid salt in the solution.

In one of embodiments, it is preferable to further add a hydroxycarboxylic acid or a salt thereof, such as citric acid or a citric acid salt during the reduction treatment. By adding a hydroxycarboxylic acid or a salt thereof during the reduction treatment, the dispersibility of platinum nanoparticles formed by the reduction is easily stabilized. Without limitation, citric acid or a citric acid salt can be preferably used.

Examples of the citrate include sodium citrate and potassium citrate. Citric acid can function as a stabilizer and a binder. Here, the binder ensures that when the solution is used by being sprayed or applied, adhesiveness between the solution and an object to which the solution is sprayed or applied is improved, and the effect of retaining the solution on the object for a longer time is promoted.

The amount of the hydroxycarboxylic acid or salt thereof added to the solution is not limited. In one of embodiments, the addition amount may be preferably 2 to 3 times the concentration of platinum ions derived from chloroplatinic acid or chloroplatinic acid salt in the solution. In one of other embodiments, the addition amount may be 0.4 to 0.6 g per 1 L of the solution.

The inorganic acid may be added during or after the reduction treatment. In one of embodiments, it is preferable to add an inorganic acid during the reduction treatment from the viewpoint of efficiently obtaining platinum nanoparticles. The pH of the solution during the reduction treatment is not limited, and is only required to be adjusted so that the solution is weekly acidic. For example, the pH of the solution may preferably be 4 to 6.

As described above, in one of embodiments, ascorbic acid can be preferably used as a reducing agent, but an undiluted solution of ascorbic acid is brown or black. Thus, from the viewpoint of avoiding coloring of the produced platinum nanoparticle solution, the amount of ascorbic acid used is preferably as small as possible. In studies on a method for suppressing time-dependent discoloration of the platinum nanoparticle solution, the present inventors have found that the time-dependent discoloration can be suppressed by adding an inorganic acid to the solution. Further, it has also been found that addition of the inorganic acid can reduce the amount of ascorbic acid used.

From such a viewpoint, in the production method of some embodiments, it is preferable that the inorganic acid is added together with the reducing agent during the reduction treatment. According to these embodiments, it may be possible to successfully perform the reduction treatment even when the amount of ascorbic acid used is reduced because the inorganic acid can also function as a reducing agent.

In one of other embodiments, from the viewpoint of enhancing the stability of dispersion of the platinum nanoparticles in the solution, an inorganic acid may be added to the solution containing the platinum nanoparticles obtained after the reduction treatment.

In one of embodiments, the amount of the inorganic acid added may be preferably 4 to 12 times the concentration of platinum ions in the solution. In one of embodiments, it is preferable that the platinum nanoparticle solution further contains citric acid from the viewpoint of stabilization as described above. In this case, the amount of the inorganic acid added may be about 2 to 6 times the amount of citric acid added. In any case, the amount of the inorganic acid added is preferably adjusted so that the pH of the platinum nanoparticle solution is 4 to 6.

In the platinum nanoparticle solution according to some of the above embodiments, the content of platinum nanoparticles is not limited, and can be adjusted by the amount of the chloroplatinic acid or chloroplatinic acid salt used during production of the platinum nanoparticle solution. By the production method of some of the above embodiments, a solution containing platinum nanoparticles at a high concentration can be easily prepared. However, even when the concentration of the platinum nanoparticles in the solution is about 0.001 to 0.1 mol/L, sufficient functions and effects can be obtained. In one of embodiments, the concentration of platinum nanoparticles in the solution may be about 0.01 mol/L.

In one of embodiments, in the platinum nanoparticle solution obtained after the reduction treatment, a part of the chloroplatinic acid or chloroplatinic acid salt used as a raw material may be contained in an original state (non-reduced state). The coexistence of the platinum nanoparticles and the chloroplatinic acid or chloroplatinic acid salt in the platinum nanoparticle solution facilitates enhancement of the stability of the platinum nanoparticles in the platinum nanoparticle solution.

In one of embodiments, the method for producing a platinum nanoparticle solution includes (i) adding a reducing agent to a solution containing platinum ions derived from chloroplatinic acid or a chloroplatinic acid salt, to subject the platinum ions to reduction treatment, and (ii) adding an inorganic acid to a solution containing platinum nanoparticles obtained by the reduction treatment. Further, the step (ii) is preferably followed by filtering the solution, and then performing stirring for 5 minutes or more if necessary. When the solution is filtered, defects such as aggregation or sedimentation of solid components in the solution are easily suppressed to enhance the storage stability of the platinum nanoparticle solution.

One of embodiments of the present invention relates to a platinum nanoparticle solution containing a solution obtained by reducing a solution containing platinum ions derived from chloroplatinic acid or a chloroplatinic acid salt, and an inorganic acid. One of other embodiments relates to a platinum nanoparticle solution obtained by mixing a solution containing platinum ions derived from chloroplatinic acid or chloroplatinic acid salt, a reducing agent containing ascorbic acid or an ascorbic acid salt, citric acid or a citric acid salt, and an inorganic acid. In these embodiments, the platinum nanoparticle solution may further contain citric acid or a citric acid salt. One of still other embodiments relates to a platinum nanoparticle solution containing platinum nanoparticles and an inorganic acid. The platinum nanoparticle solutions can be each obtained by the above-described method for producing a platinum nanoparticle solution. The platinum nanoparticle solution may further contain chloroplatinic acid or a chloroplatinic acid salt.

The platinum nanoparticle solution exhibits functions and effects such as antibacterial, deodorant and antiviral functions and effects under the catalytic action of platinum nanoparticles, and thus can form a composition that can be used for various use purposes. For example, in one of embodiments, the platinum nanoparticle solution can be preferably used as a liquid preparation such as an antibacterial agent in which a platinum nanoparticle solution is used. Hereinafter, some embodiments of the antibacterial agent will be described as an example.

### (Antibacterial agent)

The antibacterial agent may be a platinum nanoparticle solution containing platinum nanoparticles, an inorganic acid, and water. In some embodiments of the antibacterial agent, the platinum nanoparticle solution may be used in a highly concentrated (undiluted) form without being diluted, or may be used after being diluted to a desired concentration with a solvent such as water if necessary. In one of embodiments, the concentration of the platinum nanoparticles in the platinum nanoparticle solution (undiluted solution), for example, the concentration of Pt(0) in 0.265 g of a platinum nanoparticle solution in which K₂PtCl₄ is used may be about 0.05 g/L. The undiluted solution can be preferably used as an antibacterial agent after being diluted, for example, 100 to 1,000-fold with water. The dilution with water can be appropriately adjusted according to a use application.

The antibacterial agent of some of the above embodiments has excellent storage stability and can maintain good dispersibility of platinum nanoparticles even when the concentration of platinum nanoparticles in the platinum nanoparticle solution is high. Thus, some of the above embodiments can provide an antibacterial agent which resists an undesirable time-dependent of the platinum nanoparticle solution, such as deterioration of functions and effects and discoloration after storage. It is possible to obtain a better antibacterial effect with an increase in the amount of platinum nanoparticles (Pt(0)) in the solution. On the other hand, from the viewpoint of further enhancing the stability of the platinum nanoparticles in the solution, the antibacterial agent may further contain, for example, chloroplatinic acid or chloroplatinic acid. In one of embodiments, the platinum nanoparticle solution used as an antibacterial agent may contain, for example, K₂PtCl₄ at 0.2 g to 0.4 g/L.

When an antibacterial agent in which a platinum nanoparticle solution is used is applied or sprayed to a treatment surface, the antibacterial agent is dried (the solvent is evaporated) to fix the platinum nanoparticles to the treatment surface of the object. An antibacterial action is imparted to the treatment surface by the catalytic action of the platinum nanoparticles fixed to the treatment surface. In one of embodiments, deionized water can be preferably used as a solvent for diluting the platinum nanoparticle solution. If necessary, an alcohol such as ethanol is used in combination to facilitate enhancement of the ability of the antibacterial agent to dry.

The antibacterial agent may further contain other components, in addition to platinum nanoparticles, an inorganic acid and a solvent such as deionized water. In one of embodiments, it is preferable that the antibacterial agent further contains potassium iodide. Potassium iodide itself exhibits an excellent antibacterial action in a liquid form. Therefore, when potassium iodide is added to the antibacterial agent, not only the antibacterial action of the platinum nanoparticles can be exhibited after drying of the antibacterial agent, but also the antibacterial action can be exhibited before drying of the antibacterial agent (in a liquid form), so that the antibacterial action of the antibacterial agent can be further enhanced. The amount of potassium iodide added is not limited. In one of embodiments, the amount of potassium iodide added may be preferably 0.1 to 10 mass% with respect to the total mass of the antibacterial agent (platinum nanoparticle solution).

In one of embodiments, the antibacterial can be added to a coating material to form a coating material containing an antibacterial agent. The coating material may have a typical composition adopted in various applications, and can impart an antibacterial property to the coating film due to the use of the antibacterial agent. Without limitation, the coating material can be applied to structures such as buildings, electrical products such as OA equipment, daily commodities, and the like.

One of embodiments of the present invention relates to an antibacterial product in which the antibacterial agent of some of the above embodiments is applied. An antibacterial product can be produced by applying an antibacterial agent in which a platinum nanoparticle solution is used to an object to be treated, and fixing platinum nanoparticles to the surface of the object to be treated. The method for applying the antibacterial agent to the object to be treated can be carried by a method well known in the art. Without limitation, the application method may be, for example, coating or spraying. It is also possible to promote the fixation of the platinum nanoparticles by carrying out a drying step if necessary after the application of the antibacterial agent.

The object to be treated is not limited, and may be an article made from any of various materials. For example, the object to be treated may be a daily commodity made from synthetic or natural fiber, plastic, ceramic, wood, and the like. Examples of the antibacterial product include, but are not limited to, sanitary products such as masks, gauze, toothbrushes, cups, and antiseptic wipes. These antibacterial products exhibit an excellent antibacterial property, deodorant property and antiviral property under the catalytic action of platinum nanoparticles attached to the surface, and can maintain relevant functions and effects for a long period.

### Examples

Hereinafter, some embodiments of the present invention will be described by way of examples. However, the embodiment of the present invention is not limited to those described below, and includes various embodiments.

### <1> Preparation of platinum nanoparticle solution and antibacterial agent

### (Example 1)

### (1) Preparation of platinum nanoparticle solution

A solution A containing platinum ions (platinum ion concentration: 0.01 mol/L, 0.05 g/L in terms of Pt(0)) was prepared by dissolving 0.40 g of platinum (II) potassium chloride (K₂PtCl₄) in 100 mL of water (pure water). Meanwhile, 1.77 g of ascorbic acid was dissolved in 100 mL of water to prepare a solution B having an ascorbic acid concentration of 0.1 mol/L. In addition, 0.26 g of citric acid was dissolved in 100 mL of water to prepare a solution C having a citric acid concentration of 0.01 mol/L.

Next, 100 mL of the solution A, 100 mL of the solution B, and 100 mL of the solution C were added to the reaction vessel, and the mixture was stirred at room temperature (25°C) for 5 minutes.

Thereafter, dilute hydrochloric acid was added while the reaction solution was stirred, thereby obtaining a platinum nanoparticle solution (1) having a pH of 4.7.

### (2) Preparation of antibacterial agent

An antimicrobial agent (1) was prepared by adding a 1% aqueous solution of potassium iodide to 100 mL of the previously prepared platinum nanoparticle solution (1) so that the potassium iodide equivalent amount was 0.15 g/L in the solution and then mixing these.

### (Comparative Example 1)

### (1) Preparation of platinum nanoparticle solution

A platinum nanoparticle solution (C1) was prepared in just the same manner as in Example 1 except that dilute hydrochloric acid was not added in the preparation of the platinum nanoparticle solution which is described in Example 1. That is, 100 mL of the solution A, 100 mL of the solution B, and 100 mL of the solution C were added, and the mixture was stirred for 5 minutes while the temperature of the inside of the reaction vessel was maintained at room temperature (20°C), thereby obtaining the platinum nanoparticle solution (C1).

### (2) Preparation of antibacterial agent

Water was added to 100 mL of the platinum nanoparticle solution (C1) to prepare an antibacterial agent (C1). The amount of water was adjusted so that the concentration of platinum nanoparticles in the antibacterial agent (C1) was identical to that in the antibacterial agent (1) of Example 1.

### (Comparative Example 2)

### (1) Preparation of platinum nanoparticle solution

A platinum nanoparticle solution (C1) was prepared in the same manner as described in Comparative Example 1.

### (2) Preparation of antibacterial agent

To 100 mL of the platinum nanoparticle solution (C1), a 1% aqueous solution of potassium iodide was added to 0.15 g/L in the platinum nanoparticle solution in terms of potassium iodide, and mixing was performed to prepare an antibacterial agent (C2). The amounts of the platinum nanoparticle solution (C1) and the potassium iodide aqueous solution used were adjusted so that the concentration of platinum nanoparticles and the concentration of potassium iodide in the antibacterial agent (C2) were identical, respectively, to those in the antibacterial agent of Example 1.

### <2> Evaluation of storage stability (time-dependent discoloration of solution)

The antibacterial agents prepared in Example 1 and Comparative Example 1 were each placed in a transparent vessel, sealed, and stored for a long period under the same conditions (at room temperature), and a change of the solution was observed. Both the solutions were substantially transparent immediately after the start of storage. However, one week after the start of storage, the antibacterial agent of Comparative Example 1 started to color. The antibacterial agent of Comparative Example 1 became lightly brownish 4 months after the start of storage, and became more darkly brownish 6 months after the disclosure of storage. On the other hand, the antibacterial agent of Example 1 was substantially transparent 4 months, and even 6 months after the start of storage.

Fig. 1 illustrates pictures illustrating a state of time-dependent discoloration of the antibacterial agent after storage for 4 months, and Fig. 2 illustrates pictures illustrating a state of time-dependent discoloration of the antibacterial agent after storage for 6 months. In Figs. 1 and 2, picture (a) corresponds to the antibacterial agent (1) of Example 1 (with addition of dilute hydrochloric acid), and picture (b) corresponds to the antibacterial agent (C1) of Comparative Example 1 (without addition of dilute hydrochloric acid). Comparison between pictures (a) and (b) illustrated in Figs. 1 and 2 reveals that in picture (b), coloring is observed, and the color becomes darker with time. On the other hand, in picture (a), there is substantially no coloring and a transparent state is maintained even after 6 months. From this, it can be seen that dilute hydrochloric acid contributes to suppression of time-dependent discoloration.

### <3> Evaluation of properties of antibacterial agent

Various properties of the antibacterial agents prepared in each Example 1 and Comparative Examples 1 and 2 were evaluated by the following methods.

### (Antibacterial activity (1))

For the antibacterial activity of the antibacterial agent prepared in each of Example 1 and Comparative Examples 1 and 2, a test was conducted by the method of JIS Z 2801: 20105. Specifically, first, each antibacterial agent was applied to a polyethylene film, and then dried to prepare a test piece. Next, test bacteria were inoculated into the test piece, and cultured at room temperature (25°C). The test bacteria were Staphylococcus aureus (NBRC 12732) and Escherichia coli O157: H7 (Escherichia coli O157: H7 ATCC 43888). The antibacterial activity value was calculated from the number of viable bacteria immediately after the inoculation, 0.5 hours after the inoculation, and 24 hours after the inoculation. An untreated polyethylene film to which an antibacterial agent was not applied was used as a control.

Tables 1 and 2 show the results.

**[Table 1]**

| (1) Evaluation for Staphylococcus aureus | | | |
|---|---|---|---|
| Test piece (Antibacterial agent) | Normal logarithm value of number of viable bacteria (cells/cm²) | | Antibacterial activity value |
| | Immediately after inoculation | After culture for 0.5 hours | |
| Example 1 | -- | -0.04 | 3.8 |
| Comparative Example 1 | -- | 3.62 | 0.1 |
| Comparative Example 2 | -- | 3.30 | 0.4 |
| Control | 4.01 | 3.79 | -- |

| Test piece (Antibacterial agent) | Normal logarithm value of number of viable bacteria (cells/cm²) | | Antibacterial activity value |
|---|---|---|---|
| | Immediately after inoculation | After culture for 24 hours | |
| Example 1 | -- | <-0.20 | >4.9 |
| Comparative Example 1 | -- | 1.58 | 3.1 |
| Comparative Example 2 | -- | <-0.20 | >4.9 |
| Control | 4.22 | 4.72 | -- |

**[Table 2]**

| (2) Antibacterial activity against Escherichia coli O157: H7 | | | |
|---|---|---|---|
| Test piece (Antibacterial agent) | Normal logarithm value of number of viable bacteria (cells/cm²) | | Antibacterial activity value |
| | Immediately after inoculation | After culture for 0.5 hours | |
| Example 1 | -- | <-0.20 | >3.6 |
| Comparative Example 1 | -- | 3.39 | 0.0 |
| Comparative Example 2 | -- | 2.92 | 0.5 |
| Control | 3.99 | 3.45 | -- |

| Test piece (Antibacterial agent) | Normal logarithm value of number of viable bacteria (cells/cm²) | | Antibacterial activity value |
|---|---|---|---|
| | Immediately after inoculation | After culture for 24 hours | |
| Example 1 | -- | <-0.20 | >-5.9 |
| Comparative Example 1 | -- | 0.87 | 4.8 |
| Comparative Example 2 | -- | -0.10 | 5.8 |
| Control | 3.99 | 5.74 | -- |

As illustrated in Tables 1 and 2, the test piece with the antibacterial agent (1) of Example 1 is substantially comparative or superior in antibacterial activity value after culture for 24 hours to the test pieces with antibacterial agents (C1) and (C2) of Comparative Examples 1 and 2. From this, it can be seen that although the catalytic action of the platinum nanoparticles is exhibited as the antibacterial agent is dried, the addition of dilute hydrochloric acid does not deteriorate the antibacterial activity.

On the other hand, for the antibacterial activity value after culture for 0.5 hours, the test piece of Example 1 exhibited significantly better antibacterial activity over the test pieces of Comparative Examples 1 and 2. The reason why the antibacterial activity value after culture for 24 hours is higher than that after culture for 0.5 hours may be that the catalytic action of platinum nanoparticles is exhibited as the antibacterial agent is dried, and the improvement of the antibacterial activity before drying of the antibacterial agent may be ascribable to dilute hydrochloric acid.

From the above, it can be seen that by adding an inorganic acid such as dilute hydrochloric acid, excellent antibacterial activity can be exhibited even before drying of the antibacterial agent, and time-dependent discoloration can be effectively suppressed over a long period without deteriorating the original antibacterial activity after drying of the antibacterial agent.

### (Antimicrobial activity (2))

Three antibacterial agent agents (1-1), (1-2) and (1-3) differing in dilution ratio were prepared by adding deionized water to the antibacterial agent (1) prepared in Example 1. Specifically, the antibacterial agent (1-1) was diluted 50-fold, the antibacterial agent (1-2) was diluted 100-fold, and the antibacterial agent (1-3) was diluted 200-fold. The antibacterial activity of these antibacterial agents against the two types of test bacteria was evaluated in the same manner as described above. Tables 3 and 4 show the results.

**[Table 3]**

| (1) Evaluation for Staphylococcus aureus | | | |
|---|---|---|---|
| Test piece (Antibacterial agent) | Normal logarithm value of number of viable bacteria (cells/cm²) | | Antibacterial activity value |
| | Immediately after inoculation | After culture for 24 hours | |
| 1-1 | -- | <-0.20 | >4.9 |
| 1-2 | -- | -0.10 | 4.8 |
| 1-3 | -- | 3.04 | 1.6 |
| Control | 4.13 | 4.73 | -- |

**[Table 4]**

| (2) Antibacterial activity against Escherichia coli O157: H7 | | | |
|---|---|---|---|
| Test piece (Antibacterial agent) | Normal logarithm value of number of viable bacteria (cells/cm²) | | Antibacterial activity value |
| | Immediately after inoculation | After culture for 24 hours | |
| 1-1 | -- | <-0.20 | >5.4 |
| 1-2 | -- | 0.89 | 4.3 |
| 1-3 | -- | 0.97 | 4.2 |
| Control | 4.01 | 5.22 | -- |

It can be seen that excellent antibacterial activity is obtained for all the test pieces as shown in Tables 3 and 4. This demonstrates that the antibacterial agent (1) exhibits excellent antibacterial activity even after being diluted 50 to 200-fold.

From the above, it can be seen that by adding an inorganic acid, time-dependent discoloration can be effectively suppressed over a long period without deteriorating the original antibacterial activity.

### Industrial Applicability

According to the present invention, a platinum nanoparticle solution capable of resisting time-dependent discoloration can be provided. This platinum nanoparticle solution has functional properties of platinum nanoparticles such as an antibacterial property, is capable of resisting time-dependent discoloration for a long period, and thus can be preferably used for antibacterial agents, antibacterial products and the like.

## Claims

1. A method for producing a platinum nanoparticle solution, comprising: adding a reducing agent to a solution containing platinum ions derived from chloroplatinic acid or a chloroplatinic acid salt, to subject the platinum ions to reduction treatment, and adding an inorganic acid.

2. The method for producing a platinum nanoparticle solution according to claim 1, wherein the chloroplatinic acid salt contains at least one selected from the group consisting of sodium chloroplatinate, potassium chloroplatinate, platinum (II) sodium chloride, platinum (II) potassium chloride, and ammonium chloroplatinate.

3. The method for producing a platinum nanoparticle solution according to claim 1, wherein a pH of the platinum nanoparticle solution is adjusted within a range of 4 to 6 by the addition of the inorganic acid.

4. The method for producing a platinum nanoparticle solution according to any one of claims 1 to 3, wherein the inorganic acid is dilute hydrochloric acid.

5. The method for producing a platinum nanoparticle solution according to any one of claims 1 to 4, wherein the reducing agent contains at least one selected from the group consisting of ascorbic acid or an ascorbic acid salt, arginine acid or an arginine acid salt, a dicarboxylic acid, an alcohol, and a polysaccharide.

6. The method for producing a platinum nanoparticle solution according to any one of claims 1 to 5, wherein in the reduction treatment, citric acid or a citric acid salt is further added.

7. An antibacterial agent comprising: platinum nanoparticles, an inorganic acid, and water.

8. The antibacterial agent according to claim 7, wherein the inorganic acid is dilute hydrochloric acid.

9. The antibacterial agent according to claim 7 or 8, further comprising chloroplatinic acid or a chloroplatinic acid salt.

10. The antibacterial agent according to any one of claims 7 to 9, further comprising potassium iodide.

11. A coating material comprising: the antibacterial agent according to any one of claims 7 to 10.

12. An antibacterial product in which the antibacterial agent according to any one of claims 7 to 10 or the coating material according to claim 11 is applied to a daily commodity made from a material selected from the group consisting of synthetic or natural fiber, plastic, ceramic, and wood.

13. The antibacterial product according to claim 12, wherein the antibacterial product is a mask, gauze, a toothbrush, a cup, or an antiseptic wipe.
